# NEW EUROPEAN PATENT SPECIFICATION

(11) **EP 0 387 220 B2**
(45) Date of publication and mention of the opposition decision: **11.04.2001**
(45) Mention of the grant of the patent: 25.01.1995
(21) Application number: 90850064.8
(22) Date of filing: 13.02.1990
(51) Int. Cl.: A61M 16/04, A61M 16/10

(54) **A breathing device including a holder and a regenerative heat-moisture exchanger**
Beatmungsvorrichtung mit einer Halterung und einem regenerativem Wärme-Feuchtigkeits-Austauscher
Dispositif respiratoire avec support et échangeur régénératif d'humidité et de chaleur

(30) Priority: 13.02.1989 SE 8900484
(43) Date of publication of application: 12.09.1990
(73) Proprietor: ATOS MEDICAL AB, 242 22 Hörby (SE)
(72) Inventor: Lambert, Hans, S-114 25 Stockholm (SE)
(74) Representative: Petri, Stellan

(56) References cited:
- DE-A- 2 223 474
- FR-A- 2 232 330
- US-A- 3 330 271
- US-A- 3 815 754
- US-A- 4 325 366
- US-A- 4 763 645

## Description

### Technical field

The present invention relates to a breathing device for tracheotomy patients. The device comprises a holder detachably connected to the stoma in the patient's throat and has additional features.

### Background art

The need for warming and moisturizing the inspiration air in connection with the upper air passages being cut off in as a result'of tracheotomy is well known and several devices intended to fill this need have been proposed.

A known device of the above-mentioned kind is disclosed in SE-B 348 643 wherein a holder is detachably mounted to the cannula, the holder holding detachably a tube in which a regenerative heat-moisture exchanger body is disposed.

A disadvantage of the device according to the above-mentioned publication is that the device protrudes quite a bit from the patient's throat, which particularly to active people. carrying the device poses a problem with respect to the risk of their accidentally touching the device, which might then be displaced or cause pain in the patient's throat, as well from the aesthetic point of view. Further disadvantages are the relatively-complicated structure of the device and the insufficient tightness between the holder and the heat-moisture exchanger body.

A further example of a known device of the abovementioned kind is disclosed in SE-B 385 767 wherein a bandage containing, among others, a heat-moisture exchanger filter is applied directly around the stoma made in the'patient's throut.

A similar device is also earlier known from a broschure called "safe, Effective and Comfortable Humidification for the Laryngectomized Patient, Stom-Vent® from Gibeck Respiration" published in its swedish version before 1989. Also this document discloses a device where a heat moisture exhanger is connected to the skin through an adhesive.

A disadvantage of the two last-mentioned devices is that the bandage must be detached from the patient's throat every time the filter has to be changed, which sometimes has to be done several times a day. The change causes discomfort and often strain and harm to the skin, expecially if this has previously been subject to radiation treatment. Furthermore, on each change not only the filter but also other parts of the bandage must be discarded, which is uneconomical.

US-A-4 763 645 discloses a filter device including a filter holder enclosing a filter. The holder is inserted into or snapped onto the outer end of an existing tracheal tube. An end cap is detachably mounted on the holder or integrally formed with the holder.

This filter device can only be used in connection with an existing tracheal tube but not with a stoma in the throat. Further, the location of the holder within the tube makes access to the holder and filter difficult. This is a severe disadvantage because the patient wearing the filter device very often needs to remove the holder and exchange the filter therein, for instance in connection with an attack of coughing. blocking the air passage through the filter. In addition to this, the filter device does not include a heat-moisture exchanger.

DE-A- 22 23 474 refers to a heat-moisture exchanger which is connected to a tracheal tube and not to a stoma. There is no holder between the exchanger and the tube and the distance between the openings of the exchanger is longer than the extension of the holder perpendicularly thereto.

### Description of the invention

It is an object of the present invention to eliminate the disadvantages of previously known devices of the above-mentioned kind and to provide a device which protrudes only a short distance from the patient's throat, is of simple construction, provides effective sealing between the patient's throat and the heat-moisture exchange, and allows the heat-moisture exchanger to be replaced without discomfort or harm to the patient and also without parts of the device other than the heat-moisture exchanger having to be discarded.

This obiect is achieved by the device according to claim 1.

### Description of the figures

Figure 1 is a sectional view of a device according to the invention.
Figure 2 is a sectional view illustrating the connection of a speach valve to an embodiment not falling within the claimed invention.

### Preferred embodiments

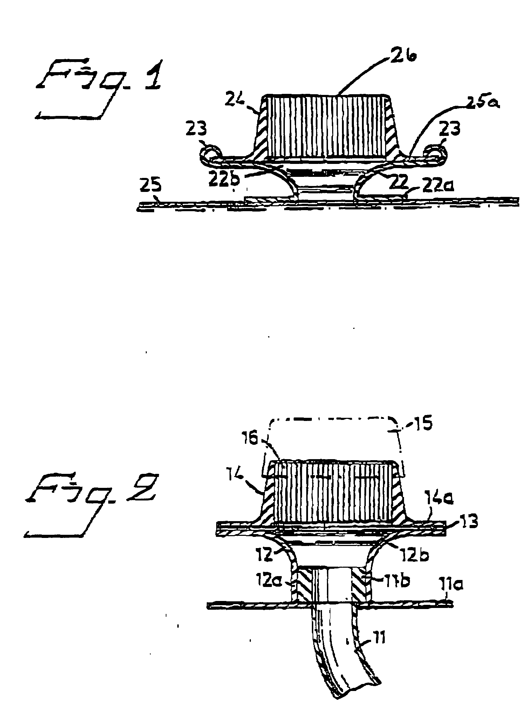

Figure 1 shows a holder 22 in the form of a funnel-shaped air-proof receptacle, said holder being provided with an opening surrounded by a circular flange 22a and a wider opening 22b opposite the first-mentioned opening and surrounded by edges parallell with the flange 22a.

A regenerative heat-moisture exchanger 24 is detachably mounted to the holder 22. The heat-moisture exchanger 24 comprises a cap-shaped plastic cover 25 containing a cylindrical, flat filter body 26 made up of helically wound strips of corrugated mini cardboard suitably impregnated with a hygroscopical material for good heat and moisture absorption from the expiration air and good heat and moisture emission to the inspiration air. The channels formed between the layers of corrugated cardboard run parallel. Instead of wound strips, the filter body 26 can be made up of cotton wool, foamed plastic or the like with substantially the same properties as the wound strips. In relation to its diameter, the filter body 26 has a low height. The filter body 26 suitably has a diameter of 24 mm and a height of 8 mm.

The heat-moisture exchanger 24 is attached to the holder 22 in one simple operation by pressing the cover 25 against the claws 23, which will then be displaced radially outwards and will snap over the flange 25a of the cover such that the latter will sealingly engage with the edges of the openin 22b. The heat-moisture exchanger 24 is removed in one simple operation by pulling it away from the holder 22. the claws 23 springing outwards thereby releasing the flange 25a.

The exchanger 24 is detachably mounted to the holder 22 by means of claws 23, as shown in Figure 1, or by adhesive means of the kind shown and described below in connection with Figure 2.

At the end of the flange 22a turned away from the exchanger 24 there is attached an annular double adhesive tape 25. On removal of a protective sheet from the end of the tape 25 turned away from the exchanger 24, the device is attached in accordance with Figure 1 around the stoma made in the patient's throat to the windpipe such that the central opening of the annular tape 25 will be right opposite the stoma in the throat and such that the tape will adhere around the stoma in the throat. Instead of the tape 25 it is possible to apply adhesive means of other suitable kind to the flange 22a.

In the device described above and depicted in the figure, the device protrude a comparatively short distance from the throat of the person carrying the same. On the holder 22 there is, a short distance between the two openings of the holder which is shorter than the extension of the holder perpendicularly thereto and is suitably less than the thickness of the exchanger 24.

Figure 2 snows a tracheal cannula 11 the outer end of which is formed as a slightly conical tube 11b with a flange 11a attached to it. A holder 12 in the form of a funnel-shaped air-proof receptacle is provided with a slightly: conical connection piece 12a, which fits onto the tube 11b of the cannula 11. On the holder 12, opposite the connection piece 12a, there is an opening 12b which is wider than the opening of the connection piece 12a, the edges of the opening 12b being perpendicular to the longitudinal axis of the connection piece 12a.

A regenerative heat-moisture exchanger 14 is attached to the holder 12. The exchanger 14 is detachably mounted to the holder 12 by adhesive means 13 between the edges of the opening 12b and the flange 14a of. the exchanger 14. The adhesive means 13 can consist of, for example a suitable glue or double adhesive tape.

Figure 2 is a diagrammatical view of a conventional speech valve 15 detachably mounted to the end of the exchanger 14 turned away from the holder 12. Although in fig. 2 the heat-moisture exchanger is connected to a tranchel cannulus, a type of connection not falling within the scope of the present invention, such a speech valve could be mounted to the heat-moisture exchanger of the device according to the embodiment.

Although some embodiments of the device according to the invention have been described above and shown on the drawings, it should be understood that the invention is not limited to said embodiments but only by the statements of the claims.

## Claims

1. A breathing device for tracheotomy patients, comprising a holder detachably connectable to a stoma in the patient's throat, the holder (22) holding a regenerative heat-moisture exchanger (24) and being an air-proof receptacle having a first opening (22a) detachably connectable to and then communicating with the stoma in the throat and a second opening (22b) situated opposite the first opening and projecting outwardly from said stoma, the heat-moisture exchanger (24), which is provided with a filter body (26) mounted to and sealingly and detachably engaging the edges of the second opening, wherein the distance between the first opening (22a) and the second opening (22b) is shorter than the extension of the holder (22) perpendicularly thereto, and wherein the detachable connection between the stoma in the throat and the holder (22) is an adhesive means (25).

2. A breathing device according to claim 1, **characterized** in that the filter body (26) is flat and preferably cylindrical, having passageways for breathing air flowing towards and from the filter body substantially in parallel with a connecting line between the two openings (22a; 22b).

3. A breathing device according to claim 1 or 2, **characterized** in that the receptacle (22) is funnel-shaped, the two openings of said receptacle being circular and the second opening (22b) being wider that the first opening (22a).

4. A breathing device according to any of the preceding claims, **characterized** in that the detachable connection between the filter body (26) and the edges of the second opening (22b) comprises resilient holding means (23) attached to said edges or an adhesive means.

5. A breathing device according to any of the preceding claims, **characterized** in that a speech valve (15) is detachably mounted to the heat-moisture exchanger (24) on the side of the same which is turned away from the holder (22).

6. A breathing device according to any of the preceding claims, **characterized** in that the distance between the stoma in the throat and the filter body (26) is less than or substantially equal to the thickness of the latter.

## Patentansprüche

1. Beatmungsvorrichtung für Patienten mit Luftröhrenschnitt umfassend einen Halter, der lösbar mit einem Atmungsloch im Hals des Patienten verbindbar ist, welcher Halter (22) einen regenerativen Wärme-Feuchtigkeitsaustauscher (24) beinhaltet, und als luftdichter Behälter ausgebildet ist, der eine erste Öffnung (22a), die mit dem Atmungsloch im Hals lösbar verbindbar ist und dann mit diesem in Verbindung steht, sowie eine zweite Öffnung (22b) aufweist, die an der zur ersten Öffnung entgegengesetzten Seite liegt und vom genannten Atmungsloch nach außen vorragt, wobei der mit einem Filterkörper (26) versehene Wärme-Feuchtigkeitsaustauscher (24) montiert ist an die Ränder der zweiten Öffnung und dicht und lösbar in die Ränder der zweiten Öffnung eingreift, wobei der Abstand zwischen der ersten Öffnung (22a) und der zweiten Öffnung (22b) kleiner ist als das Ausmaß des Halters (22) senkrecht dazu und wobei die lösbare Verbindung zwischen dem Atmungsloch in dem Hals und dem Halter (22) ein Klebemittel (25) ist.

2. Beatmungsvorrichtung nach Anspruch 1, dadurch gekennzeichnet, daß der Filterkörper (26) flach und vorzugsweise zylindrisch ist sowie Durchgänge für die Atemluft aufweist, die zum und vom Filterkörper im wesentlichen parallel zu einer Verbindungsleitung zwischen den zwei Öffnungen (22a, 22b) strömt.

3. Beatmungsvorrichtung nach Anspruch 1 oder 2, dadurch gekennzeichnet, daß der Behälter (22) trichterförmig ist, wobei die beiden Öffnungen des Behälters kreisförmig sind und die zweite Öffnung (22b) größer als die erste Öffnung (22a) ist.

4. Beatmungsvorrichtung nach einem der vorhergehenden Ansprüche, dadurch gekennzeichnet, daß die lösbare Verbindung zwischen dem Filterkörper (26) und den Rändern der zweiten Öffnung (22b) federnde an den Rändern angebrachte Halteorgane (23) oder ein Klebemittel umfaßt.

5. Beatmungsvorrichtung nach einem der vorhergehenden Ansprüche, dadurch gekennzeichnet, daß auf der vom Halter (22) abgewendeten Seite des Wärme-Feuchtigskeitsaustauschers (24) ein Sprachventil (15) lösbar angebracht ist.

6. Beatmungsvorrichtung nach einem der vorhergehenden Ansprüche, dadurch gekennzeichnet, daß der Abstand zwischen dem Atmungsloch im Hals und dem Filterkörper (26) kleiner als oder im wesentlichen gleich der Dicke des letzteren ist.

## Revendications

1. Dispositif respiratoire pour des patients trachéotomisés, comprenant un support se raccordant de manière détachable à une ouverture dans la gorge du patient, le support (22) portant un échangeur de chaleur et d'humidité régénérateur (24) et se présentant comme un réceptacle étanche à l'air ayant une première ouverture (22a) se raccordant de manière détachable à, et communicant alors avec, l'ouverture dans la gorge, et une deuxième ouverture (22b) située à l'opposé de la première ouverture et saillant vers l'extérieur de ladite ouverture dans la gorge, l'échangeur de chaleur et d'humidité (24), qui est muni d'un corps de filtre (26, 16), monté et s'engageant de manière étanche et détachable avec les bords de la deuxième ouverture, appareil selon lequel la distance entre la première ouverture (22a) et la deuxième ouverture (22b) est plus courte que l'extension du support (22) perpendiculairement à celle-ci, et selon lequel le raccord détachable entre l'ouverture dans la gorge et le support (22) est un moyen adhésif (25).

2. Dispositif respiratoire selon la revendication 1, caractérisé en ce que le corps de filtre (26) est plat et de préférence cylindrique, ayant des passages pour que de l'air de respiration s'écoule vers et depuis le corps de filtre substantiellement en parallèle avec un conduit de liaison entre les deux ouvertures (22a, 22b).

3. Dispositif respiratoire selon la revendication 1 ou 2, caractérisé en ce que le réceptacle (22) a une forme d'entonnoir, les deux ouvertures dudit réceptacle étant circulaires et la deuxième ouverture (22b) étant plus large que la première ouverture (22a).

4. Dispositif respiratoire selon l'une quelconque des revendications précédentes, caractérisé en ce que le raccord détachable entre le corps de filtre (26) et les bords de la deuxième ouverture (22b) comprend des moyens de maintien élastiques (23) fixés auxdits bords, ou un moyen adhésif.

5. Dispositif respiratoire selon l'une quelconque des revendications précédentes, caractérisé en ce qu'une soupape de parole (15) est montée de manière détachable sur l'échangeur de chaleur et d'humidité (24) sur le côté de celui-ci qui est opposé au support (22).

6. Dispositif respiratoire selon l'une quelconque des revendications précédentes, caractérisé en ce que la distance entre l'ouverture dans la gorge et le corps de filtre (26) est inférieure à ou substantiellement égale à l'épaisseur de ce dernier.
